# EUROPÄISCHE PATENTSCHRIFT

(11) **EP 1 542 617 B1**
(45) Veröffentlichungstag und Bekanntmachung des Hinweises auf die Patenterteilung: **14.09.2016**
(21) Anmeldenummer: 03765067.8
(22) Anmeldetag: 21.07.2003
(51) Int. Cl.: A61F 2/92, A61F 2/90, A61F 2/915, A61B 17/12, A61B 17/221

(54) **Kombination eines medizinischen Implantats und eines Führungsdrahts**
Combination of a medical implant and a guide wire
Combination d'un implant médical et un fil de guidage

(30) Priorität: 19.07.2002 DE 10233085
(43) Veröffentlichungstag der Anmeldung: 22.06.2005
(60) Teilanmeldung: 11187263.6
(73) Patentinhaber: Dendron GmbH, 44799 Bochum (DE)
(72) Erfinder: HENKES, Hans, 44797 Bochum (DE); FLESSER, Achim, 40822 Mettmann (DE); KONTEK, Ronald, 44651 Herne (DE); SPEDER, Jürgen, 44807 Bochum (DE); BODENBURG, Ralph, 44801 Bochum (DE)
(74) Vertreter: Hoffmann Eitle
(86) Internationale Anmeldenummer: PCT/EP2003/007926
(87) Internationale Veröffentlichungsnummer: WO 2004/008991

(56) Entgegenhaltungen:
- WO-A-01/32099
- WO-A-96/28116
- DE-A- 10 010 840
- DE-A- 19 703 482
- US-B1- 6 264 686

## Beschreibung

Die Erfindung betrifft ein medizinisches Implantat, welches zur Einnahme einer übergeordneten Struktur am Implantationsort vorgeformt ist, während der Einführung jedoch in einer volumenreduzierten Form vorliegt.

Die Erfindung betrifft weiterhin die Kombination dieses Implantats mit einem Führungsdraht sowie ein System zur Verwendung derartiger Implantate bei der Behandlung von Aneurysmen oder anderer vaskulärer Fehlbildungen.

Es ist bekannt, Gefäßverengungen (Stenosen) durch den Einsatz von Stents (Gefäßendoprothesen, Gefäßstützen) zu behandeln, welche in den stenotischen Bereich eingeschoben werden und dort durch ihre Eigensteifigkeit das Gefäßlumen offen halten. Auch ist bekannt, solche Stents zum Verschluß von Gefäßwandaussackungen (Aneurysmen) oder Fisteln einzusetzen.

Herkömmlicherweise werden dabei ballondilatierbare Stents eingesetzt. Diese werden zur Einbringung in nicht-dilatiertem Zustand auf einen nichtexpandierten Ballon aufgekrimpt, über ein Kathetersystem an die zu behandelnde Stelle geführt und dort durch Expansion des Ballons dilatiert und so im Gefäß verankert. Da zur Einführung ballondilatierbarer Stents keine aufwendigen Stütz- und Überhüllen notwendig sind, können diese auch in sehr feine Gefäße eingeführt werden. Problematisch ist jedoch, daß sie aufgrund ihrer plastischen Verformbarkeit durch Druckeinwirkung von außen einfach zusammengedrückt werden können. Ein weiterer Nachteil besteht darin, daß sie zur Verankerung mittels Beaufschlagung mit hohem Druck zunächst über den Umfang ausgedehnt werden müssen, den sie letztlich einnehmen. Diese Aufweitung über den notwendigen Umfang birgt die Gefahr einer Gefäßverletzung, was die Bildung von Thromben nach sich ziehen kann.

Ein weiterer Nachteil dieser herkömmlichen ballondilatierbaren Stents beruht darauf, daß sie aufgrund ihrer Struktur nicht einfach durch einen gelegten Mikrokatheter hindurch an den Bestimmungsort geschoben werden können, sondern in einem speziell dafür ausgelegten Mikrokatheter in dessem distalen Bereich angeordnet werden müssen, um über einen sogenannten Pusher an den Implantationsort befördert zu werden. Dies macht die dazu benötigte Kathetertechnik aufwendig und schwer handhabbar. Ein weiteres Problem ist, daß ein einmal ausgebrachter Stent, wenn überhaupt, nur sehr schwer relokalisiert oder zurückgeholt werden kann. Ein einmal dilatierter Stent kann, wenn er falsch platziert wurde, in der Regel nicht mehr in seiner Position verändert oder wieder entfernt werden.

Es ist ferner bekannt, selbstexpandierende Stents einzusetzen, die aus Formgedächtnismaterialien gefertigt sind. Diese besitzen eine geflechtartige Struktur und werden zunächst in kollabiertem Zustand durch einen Katheter an den Zielort geführt, wo sie sich in Folge der Temperaturveränderung (thermischer Formgedächtniseffekt) oder durch den Wegfall des durch den Katheter ausgeübten mechanischen Zwanges (Superelastizität) aufweiten. Solche Stents weisen ebenfalls den Nachteil auf, daß die zur Einführung notwendigen Vorrichtungen relativ aufwendig und platzintensiv sind. So ist bei den bekannten superelastisch expandierbaren Stents stets eine Stütz- und Überhülle nötig, was einen relativ großen Katheterumfang bedingt und die Einbringung solcher Stents durch einen bereits gelegten Katheter ebenfalls ausschließt.

Für den Einsatz in besonders kleinlumigen intrakraniellen Gefäßen ist es ferner bekannt, Stents aus Formgedächtnismaterial einzusetzen, die zunächst als lang gestrecktes Filament vorliegen und erst bei Austritt durch den Katheter durch die Temperaturveränderung oder den Wegfall des zuvor aus dem Katheter ausgeübten Zwangs die röhrenförmige Struktur eines Stents annehmen.

Aus der DE 197 03 482 A1 ist es bekannt, zur Behandlung von Aneurysmen und dergleichen einen Stent aus zwei lang gestreckten Filamenten einzusetzen, welche durch den mechanischen Zwang des Stranges spannungsinduziert in der lang gestreckten Form gehalten werden und nach Wegfall dieses Zwanges, bei Herausschieben aus dem Katheter, die eigentliche Stent-Form einnehmen. Hierdurch wurde erstmals der Einsatz von Stents mit Formgedächtniseigenschaften auch in sehr kleinlumigen Gefäßen in Folge der intrakraniellen und zerebralen Gefäßäste möglich.

Diese Stents, die für gewisse Einsatzzwecke gut geeignet sind, weisen aber eine Reihe von Nachteilen auf, darunter ihre relativ schwere Verschiebbarkeit im und die fehlende Rückführmöglichkeit in den Katheter, letzteres bei Fehlplatzierung. Auch ist der Stent, aufgrund seiner sehr filigranen Struktur, wenig geeignet, Aussackungen und Fisteln in den Gefäßen so abzudecken, dass darin platzierte Okklusionsmittel zurückgehalten werden.

Als weiterer Stand der Technik ist das Dokument WO 96/28116 A1 bekannt, das eine röhrenförmige Prothese betrifft. Das röhrenförmige Bauteil ist aus maschenartigem Metallgewebe aufgebaut. In einer Ausführungsform kann das röhrenförmige Bauteil ein aus einem biokompatiblen Material bestehendes Gewebe umfassen, das konzentrisch aufgerollt ist.

Angesichts der mit dem Stand der Technik verbundenen Nachteile besteht die Aufgabe der Erfindung in der Bereitstellung von Implantaten, welche auch in kleiniumige intrakranielle Gefäße durch herkömmliche Mikrokatheter hindurch eingebracht werden können, gut positionierbar und repositionierbar sind, bei Bedarf in den Mikrokatheter zurückgeführt werden können, und geeignet sind, Gefäßaussackungen und Fisteln so zu überbrücken, dass diese mit Okklusionsmitteln gefüllt werden können. Zudem wäre es wünschenswert, über Implantate zu verfügen, die sich dem Gefäßkaliber relativ frei anpassen können, d.h. nicht auf ein besonderes Gefäßkaliber hin gefertigt sind.

Diese Aufgabe wird erfindungsgemäß durch eine Kombination eines medizinischen Implantats und eines Führungsdrahts gemäß Anspruch 1 gelöst.

Anders ausgedrückt besteht das erfindungsgemäße Implantat aus einem flächigen Gegenstand, der aufgrund der ihm aufgeprägten überlagerten Struktur die Form eines aufgeschlitzten Rohres oder Schlauches einnimmt, wobei sich die freien Schenkel vorzugsweise überlappen. In seiner volumenreduzierten Form liegt er weiter eingerollt vor, d. h. der Durchmesser des Implantates in seiner volumenreduzierten Form ist gegenüber dem der überlagerten Struktur deutlich verringert. Nach Freisetzung des Implantates trachtet dieses, die ihm aufgeprägte Struktur einzunehmen und weitet sich aus, soweit es das das Implantat umgebende Gefäß zulässt. Diese Aufweitung nach Art einer sich aufweitenden Spiralfeder führt zu einer automatischen Anpassung des Implantates an das Gefäßkaliber bzw. -lumen dergestalt, daß es in Gefäßen unterschiedlichen Kalibers eingesetzt werden kann. Bei engen Gefäßen ergibt sich so eine relativ starke Überdeckung der beiden freien Schenkel, bei weiten Gefäßen eine geringe Überdeckung bzw. ein freibleibender Spalt, der gegebenenfalls, bei Gefäßverzweigungen, sogar gewünscht sein kann.

Das Implantat selbst hat eine Maschenstruktur aus untereinander verbundenen Stegen oder Filamenten. Stege treten auf, wenn das Implantat geschnittene Strukturen aufweist, wie sie beispielsweise bei Coronarstents häufig verwandt werden, eine Maschenstruktur aus Filamenten dann, wenn die Implantate als Matten mit gewirkter oder geflochtener Struktur oder aus miteinander verschweißten Einzelfilamenten vorliegen.

Wesentlich für die Erfindung ist, daß das Implantat eine Flächenstruktur ist, die zu einem längs offenen schlauchförmigen Gebilde gerollt ist, das sich an die Wandung des damit zu beaufschlagenden Gefäßes eng anlegen kann.

Die einseitig zu einer sich verjüngenden Struktur in einen Verbindungspunkt zusammenlaufenden Stege oder Filamente erlauben es, das noch mit einem Führungsdraht verbundene Implantat im Falle einer Fehlplatzierung oder unzureichenden Anpassung an den Implantationsort problemlos in den Katheter zurückzuziehen, um es gegen ein anderes Implantat auszutauschen oder es nach Repositionierung des Katheters erneut zu implantieren. Durch die sich verjüngende Struktur rollt sich das Implantat bei Eintritt in den Mikrokatheter enger zusammen und nimmt erneut seine volumenreduzierte Form an, wobei die Zugkraft des Führungsdrahtes und die vom Katheterrand ausgeübten Kräfte zusammenwirken.

Im Katheter selbst liegt das Implantat in seiner volumenreduzierten Form, etwa wie aufgerollter Maschendraht vor. Unter der Einwirkung des Führungsdrahtes kommt bei Anwendung von Schubkräften eine axiale Kompression hinzu, bei Entspannung und Einnahme der überlagerten Struktur gegebenenfalls eine geringe Längenkontraktion. Die bei dilatierbaren Stents beobachtete Längenkontraktion tritt bei den erfindungsgemäßen Stents allerdings nur unwesentlich auf.

Der Verbindungspunkt des medizinischen Implantates am Ende der verjüngten Struktur ist gleichzeitig der Anknüpfungspunkt an den Führungsdraht, entweder direkt oder über ein Verbindungselement. Im Falle einer geschnittenen oder Strickmetallfolie handelt es sich dabei um den Punkt, in dem die Stege des Implantates zusammenlaufen. Bei einer Maschenstruktur aus einzelnen Filamenten laufen dort wenigstens zwei Filamente zusammen und sind durch Verschweißen oder Verkrimpen miteinander verbunden.

In der Regel ist der Verbindungspunkt gleichzeitig ein Verbindungselement bzw. ein Teil desselben, welches nach der Ablösung des Implantates vom Führungsdraht am Implantat verbleibt. Es kann zweckmäßig sein, diesen Verbindungspunkt in einer Platinspirale anzuordnen oder über eine Platinspirale mit einem Verbindungselement zu verbinden, welche gleichzeitig bei der Positionierung als röntgendichter Marker dienen kann. Besonders bevorzugt sind elektrolytisch korrodierbare Verbindungselemente, wie sie beispielsweise in der DE 100 10 840 A1 beschrieben sind. Derartige Verbindungselemente ermöglichen es, das Implantat nach seiner korrekten Positionierung durch kurzfristige Stromeinwirkung von beispielsweise 10 bis 60 s vom Führungsdraht abzulösen.

Wie bereits dargestellt, besteht das medizinische Implantat aus einem flächigen Gebilde, das sich aufgrund der ihm vorgegebenen überlagerten Struktur zu einem Schlauch zusammenrollt. Vorzugsweise kommt es dabei zu einer wenigstens geringen Überdeckung der freien Schenkel des Implantates.

Das Implantat selbst kann aus einer Folie bestehen, die, beispielsweise durch Lasertechnik, mit den entsprechenden Stegmustern versehen ist. Die Stegbreite beträgt beispielsweise 0,05 bis 0,2 mm. Die Fertigungstechnik ist die gleiche, wie sie bei röhrenförmigen Coronarstents angewandt wird. Alternativ dazu können auch Streckmetallfolien eingesetzt werden, wobei die Stegbreiten sich in der gleichen Größenordnung bewegen. Hierbei ist es bevorzugt, die Folie anschließend zu glätten, so daß sich alle Stege in einer Ebene befinden. Die Foliendicke liegt in der Regel im Bereich von 0,02 bis 0,2 mm, Die größeren Folienstärken erlauben auch den Einsatz in anderen Bereichen, beispielsweise als Coronarstents oder in anderen Körperbereichen, etwa im Gallengang oder im Harnleiter.

Die Maschenweite liegt in der Regel im Bereich von 0,5 bis 4 mm und kann innerhalb eines Implantates variieren. Gleiches gilt auch für die Stegbreite. So ist es im allgemeinen bevorzugt, im Bereich der Verjüngung größere Maschenweiten und -längen und/oder größere Stegbreiten oder stärkere Filamente zu verwenden. Im Bereich der sich verjüngenden Struktur ist im allgemeinen keine besonders große Stützwirkung und Abdeckung der Gefäßwand erforderlich, andererseits eine erhöhte Zug- und Schubfestigkeit.

Bei der Verwendung von auf Filamenten aufbauenden Maschenstrukturen können gewirkte oder gestrickte Strukturen verwendet werden wie auch durch Verschweißung verbundene Filamente. Die Filamentstärken liegen im allgemeinen im Bereich von 0,01 bis 0,1 mm und vorzugsweise bei 0,02 bis 0,076 mm. Für die Maschenweiten gilt das bereits oben gesagte.

Soweit die Maschenstruktur aus miteinander verschweißten einzelnen Filamenten besteht, wird vorzugsweise eine Laserschweißtechnik angewandt. Bei einem Maschengeflecht einzelner Filamente werden an und für sich bekannte Flecht-, Wirk- oder Stricktechniken angewandt, wie sie beispielsweise aus der Maschendrahtfertigung oder der Textiltechnik bekannt sind. Dabei sind besonders bevorzugt Maschengeflechte, die eine Wirkstruktur aufweisen, die herstellungsbedingt zu eingerollten Rändern führt, weil auf diese Art und Weise die erforderliche überlagerte Struktur über das Wirkverfahren erzeugt werden kann. Besonders bevorzugt ist eine dem Textilfachmann als "Fluse" bekannte Wirkstruktur.

Ein besonderer Vorteil der erfindungsgemäßen medizinischen Implantate gegenüber herkömmlichen aufweitbaren Stents besteht darin, daß bei der Anpassung an das zu beaufschlagende Gefäß keine Längenkontraktion mehr stattfindet. Die längs offene Struktur mit der vorgegebenen "Wicklung" hat keinerlei Einfluß auf die Längenausdehnung des Stents. Die Folienstrukturen selbst haben sich auch bei der Positionierung unter Schub- und Zugwirkung als ausgesprochen dimensionsstabil erwiesen. Entsprechendes - gilt für die Wirkstruktur und die Maschenstruktur aus verschweißten einzelnen Filamenten.

Als Filamente können auch solche verwandt werden, die aus einem Geflecht von Einzellitzen bestehen, also ein Seil darstellen. Geflechte aus 12 bis 14 Litzen mit einer Gesamtstärke von 0,02 mm haben sich als brauchbar erwiesen.

Soweit den Implantaten die überlagerte Struktur nicht aufgrund der Wirk-, Strick- oder Flechttechnik aufgegeben werden kann, ist der Einsatz von Materialien sinnvoll, die über Formgedächtniseigenschaften verfügen. Solche Legierungen sind beispielsweise Titan und Nickel enthaltende Legierungen, die unter der Bezeichnung Nitinol bekannt geworden sind, Eisenbasis- oder Kupferbasislegierungen. Formgedächtniseigenschaften können auf einer spannungsinduzierten martensitischen Transformation beruhen wie auch auf einer temperaturinduzierten martensitischen Transformation oder Kombinationen der beiden.

Als Materialien für die Filamente kommen insbesondere auch Platin, Platinlegierungen, Gold und nichtrostender Stahl in Frage. Im allgemeinen können alle Dauerimplantatwerkstoffe der Medizintechnik eingesetzt werden, die über die benötigten Eigenschaften verfügen.

Wie schon erwähnt, weisen die erfindungsgemäßen Implantate insbesondere auch röntgendichte Marker auf, die die Überwachung der Positionierung und Implantation erlauben. Als solche Marker können proximal, insbesondere am Verbindungspunkt der Stege bzw. Filamente angeordnete Spiralen dienen. Vorzugsweise befinden sich röntgendichte Marker auch am distalen Ende des Implantates, insbesondere in Form von in die Maschenstruktur eingearbeiteten oder an die Maschenstruktur angehängten Platin- oder Platin/Iridiumelementen. Insbesondere können die Maschen des erfindungsgemäßen Implantates distal mit einer Öse versehen sein oder zu einer Öse auslaufen, in der sich das Markerelement flächeneben angeordnet befindet.

Die Erfindung betrifft ferner die Kombination des vorstehend beschriebenen Implantates mit einem Führungsdraht, der das Implantat an seinem distalen Ende lösbar angeordnet aufweist. Die Lösbarkeit wird insbesondere durch ein durch Stromeinwirkung korrodierbares Element herbeigeführt, wie es aus dem Stand der Technik bekannt ist. Bei dem Führungsdraht handelt es sich um einen ansonsten üblichen Führungsdraht, der geeignet ist, sowohl das Implantat durch einen Katheter hindurch zum Einsatz zu schieben und auch im Falle der Fehlpositionierung in den Katheter hinein zurückzuziehen. Naturgemäß kann die Korrosionsstelle auch im Bereich des Führungsdrahtes selbst liegen oder auf einer an und für sich bekannten mechanischen oder thermischen Ablösetechnik beruhen.

Schließlich betrifft die Erfindung auch ein System zur Verwendung bei der Behandlung von Aneurysmen oder anderer vaskulärer Fehlbildungen, das über einen ersten Mikrokatheter verfügt, einen ersten Führungsdraht zur Platzierung des ersten Mikrokatheters, einen zweiten Führungsdraht, der dazu bestimmt ist, das Implantat durch den ersten Mikrokatheter zu transportieren und zu platzieren sowie das Implantat, welches lösbar am distalen Ende des zweiten Führungsdrahtes angeordnet ist. Die eingerollte Struktur des Implantates und die Kombination mit dem Führungsdraht ermöglicht es, nach Platzierung des ersten Mikrokatheters den ersten Führungsdraht zu entfernen und den zweiten Führungsdraht mit dem Implantat einzuführen und zu handhaben. Bisherige Implantate dieser Art wurden stets mit einer sogenannten Pusher-Technik gesetzt, bei denen die Zurückholung des Implantates in der Regel ausgeschlossen war.

Gemäß einer bevorzugten Ausführungsform weist das System zusätzlich einen zweiten Mikrokatheter auf, der dazu ausgelegt und bestimmt ist, den zweiten Führungsdraht mit dem Implantat in sich verschiebbar aufzunehmen und durch den ersten Mikrokatheter zum Einsatz zu führen. Die Gleitfähigkeit fördernde Beschichtungen des zweiten Mikrokatheters können die Handhabung erleichtern.

Der erste Mikrokatheter ist ein an und für sich üblicher Mikrokatheter, wie er beispielsweise mit einem Durchmesser/Kaliber von 0,51 mm (20 mil) bis 0,36 mm (14 mil) in der Neuradiologie weit verbreitet ist.

Das System kann ferner übliche elektrische Einrichtungen zur elektrolytischen Ablösung des Implantats vom Führungsdraht an einer dafür vorgesehenen Ablösestelle aufweisen.

Die Erfindung wird durch die nachfolgend beschriebenen Abbildungen näher beschrieben. Es zeigen:
- Fig. 1:: Ein erfindungsgemäßes Implantat mit einer Wabenstruktur,
- Fig. 2:: eine weitere Ausführungsform eines erfindungsgemäßen Stents mit einer Wabenstruktur;
- Fig. 3:: eine dritte Ausführungsform eines erfindungsgemäßen Stents mit einer Wabenstruktur;
- Fig. 4:: eine Wirkstruktur, wie sie für erfindungsgemäße Implantate in Frage kommt;
- Fig. 5:: einen erfindungsgemäßen Stent samt Führungsdraht und Katheter;
- Fig. 6:: schematisch ein erfindungsgemäßes Implantat in seiner überlagerten und in seiner volumenreduzierten Form;
- Fig. 7:: ein Markerelement, wie es erfindungsgemäß zum Einsatz kommen kann;
- Fig. 8:: schematisch zwei Ablösestellen, über die das erfindungsgemäße Implantat lösbar mit einem Führungsdraht verbunden werden kann.

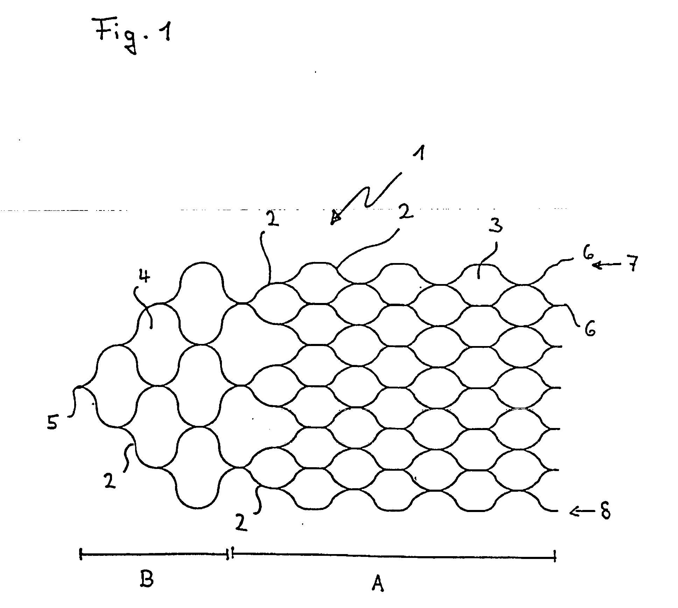

Das Implantat gemäß Figur 1 besteht aus einer Maschen- oder Wabenstruktur, die im vorliegenden Fall aus einer Mehrzahl von Filamenten besteht, die mit Hilfe von Laserschweißtechnik miteinander verbunden wurden. Das Implantat gliedert sich in einen eigentlichen funktionellen Teil A und die sich verjüngende proximale Struktur B, die sich u. a. durch eine unterschiedliche Maschengröße unterscheiden. Zur Wahrnehmung ihrer Rückhaltefunktion sind im funktionellen Teil A die Maschen 3 relativ eng, so daß sie geeignet sind, in einem Aneurysma angeordnete Occlusionsspiralen zurückzuhalten. Im sich verjüngenden proximalen Teil B des Implantats herrscht eine weite Maschenstruktur 4 vor, die auf eine minimale Occlusionswirkung hin optimiert wurde. Im Bereich der sich verjüngenden Struktur 2 weisen die Filamente vorzugsweise eine größere Stärke auf, um die im Verbindungspunkt 5 ansetzenden Schub- und Zugkräfte des Führungsdrahts beim Setzen des Implantats auf den funktionellen Teil A besser übertragen zu können. Filamentstärken im funktionellen Teil A liegen im allgemeinen in einer Größenordnung von 0,02 bis 0,076 mm im Teil B bei 0,076 mm und darüber.

Der proximale Teil B bildet im Verbindungspunkt 5 vorzugsweise einen Winkel von 45° bis 120° aus, insbesondere etwa 90°. Die Filamentstärke (bzw. Stegbreite) wie auch die Maschengröße und -form kann in weiten Bereichen variieren, je nach Anforderung an Stabilität, Flexibilität und dergleichen. Es versteht sich, daß sich auch der proximale Teil an die Gefäßwandung anlehnt und den Blutfluß im Gefäß nicht behindert.

Am distalen Ende laufen die Filamente 2 zu einer Reihe von "Schwänzen" 6 aus, die geeignet sind, Platinmarker zu tragen, die die Positionierung des Implantats erleichtern.

In seiner überlagerten Struktur ist das Implantat 1 so aufgerollt, daß die Ränder 7 und 8 einander zumindest nahe sind, vorzugsweise sogar im Randbereich überlappen. In der volumenreduzierten Form ist das Implantat 2 nach Form einer Maschendrahtrolle soweit aufgerollt, daß die gebildete Rolle problemlos in einen Mikrokatheter eingeschoben und darin bewegt werden kann. Nach Freisetzung aus dem Mikrokatheter springt die aufgerollte Struktur auf und trachtet die ihm aufgeprägte überlagerte Struktur einzunehmen und legt sich dabei an die Innenwand des zu beaufschlagenden Gefäßes eng an, wobei eine dort vorhandene Fistel, Gefäßabzweigung oder ein Aneurysma oberflächlich abgedeckt werden. Der Grad des "Aufrollens" wird dabei vom Gefäßvolumen bestimmt; in engeren Gefäßen kommt es zu einer stärkeren Überdeckung der Ränder 7 und 8 des Implantats 1, in weiten Gefäßen zu einer geringen Überdeckung oder sogar Unterdeckung, wobei darauf geachtet werden muß, daß das Implantat noch eine Restspannung aufweist.

Als Materialien kommen Legierungen mit Förmgedächtniseigenschaften in Frage. Das fertige Produkt wird bei den für das Material üblichen Temperaturen getempert, so daß die aufgeprägte Struktur fixiert wird.

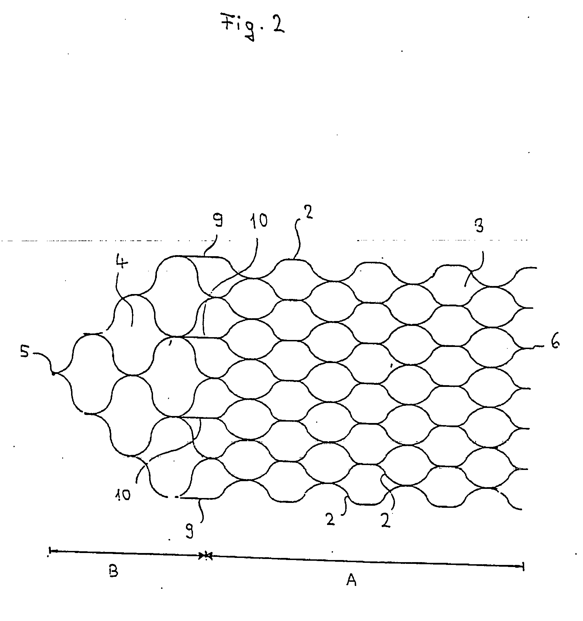

Figur 2 zeigt eine weitere Ausführungsform eines erfindungsgemäßen Stents 1 mit der bereits zuvor beschriebenen Wabenstruktur, bei der der sich verjüngende proximale Teil B durch zusätzliche Filamente 9 im peripheren Bereich 10 und im zentralen Bereich mit dem funktionellen Teil A verbunden ist. Die zusätzlichen Filamente 9 und 10 bewirken eine gleichmäßigere Übertragung von Zug- und Schubkräften von der proximalen Struktur B auf den funktionellen Teil A, so daß insbesondere bei einer gegebenenfalls notwendigen Repositionierung des Stents durch Zurückziehen in den Mikrokatheter die Zugkräfte besser übertragen werden können. Hierdurch erleichtert sich das erneute Einrollen des Stents. Gleichermaßen wird auch das Übertragen von Schubkräften beim Ausfahren und Setzen des Stents erleichtert und ein sanftes Absetzen ermöglicht. Im übrigen bezeichnen gleiche Ziffern gleiche Positionen.

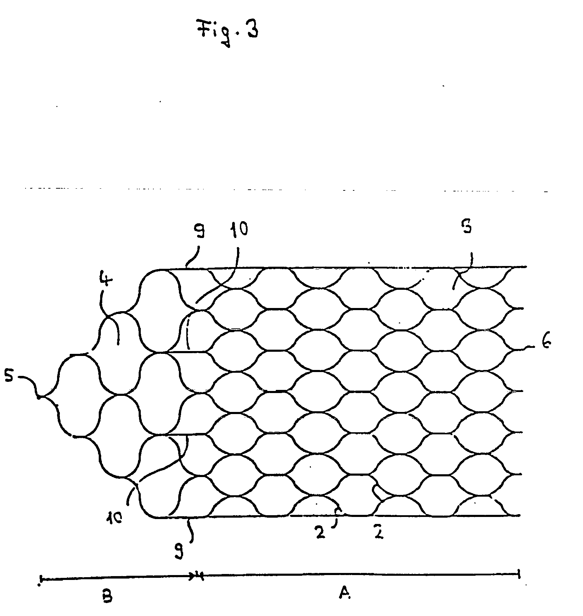

Figur 3 zeigt eine weitere Ausführungsform eines erfindungsgemäßen Stents 1 mit einer Wabenstruktur, bei der die Ränder 7 und 8 von im wesentlichen gradlinig verlaufenden Filamenten 9 gebildet werden. Gemäß dieser Ausführungsform wird der über den Punkt 5 vom Führungsdraht ausgeübte Schub oder Druck sehr direkt auf die Ränder 7 und 8 des funktionellen Stentteils A übertragen, was den in Bezug auf Figur 2 geschilderten Effekt weiter verstärkt.

Die Ausführungsform gemäß Figur 3 kann, wie auch diejenigen von Figur 1 und 2, auf einer geschnittenen Folie beruhen, d. h. die einzelnen Filamente 2, 9 und 10 sind durch einzelne Stege ersetzt, die bei einer mit Schneidtechnik bearbeiteten Folie stehen gelassen wurden. Laserschneidtechniken zur Herstellung von Stents mit einer Röhrenstruktur sind bekannt und vielfach beschrieben. Die Bearbeitung einer Folie zur Erzeugung eines für einen Stent geeigneten Musters verläuft entsprechend. Das Aufprägen der überlagerten Struktur erfolgt auf gleiche Art und Weise, wie auch bei der Filamentausführung.

Mit Schneidtechnik bearbeitete Folien werden vorzugsweise elektrochemisch nachbearbeitet, um Grate und andere Unregelmäßigkeiten zu entfernen, eine oberflächliche Glättung herbeizuführen und Kanten zu runden. Derartige Bearbeitungsverfahren elektrochemischer Natur sind dem Fachmann bekannt und werden in großem Umfang in der Medizintechnik bereits eingesetzt. Im Zusammenhang ist anzumerken, daß die erfindungsgemäßen Stents, die auf zweidimensionalen Geometrien beruhen und denen eine dreidimensionale Struktur erst später aufgeprägt wird, grundsätzlich einfacher zu fertigen und zu bearbeiten sind, als die klassischen "Röhren"stents, die bereits bei der Fertigung in einer dreidimensionalen Struktur vorliegen und entsprechend aufwendiger Bearbeitungsverfahren/-vorrichtungen bedürfen.

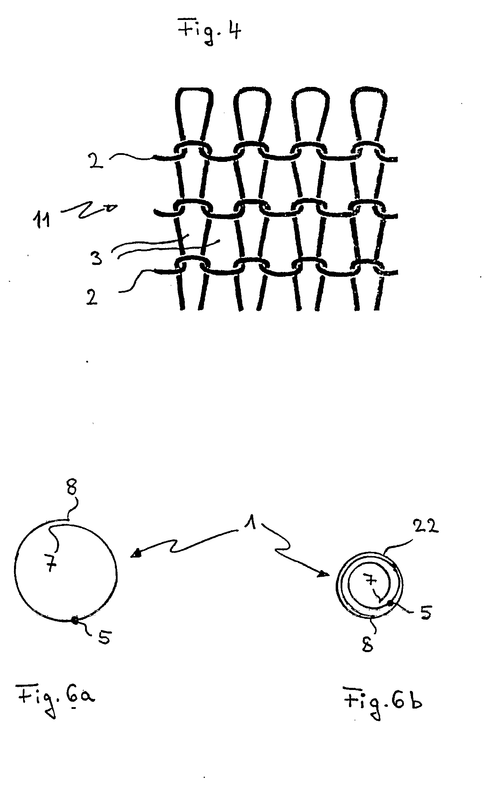

Wie zuvor dargelegt, kann die Maschenstruktur des erfindungsgemäßen Implantats aus einem Geflecht einzelner Filamente bestehen. Eine derartige Wirkstruktur ist in Figur 4 dargestellt, in der die einzelnen Filamente 2 nach Art eines rechts links Gestrickes zu einzelnen Maschen 3 einer Maschenstruktur 11 verwirkt sind. Derartige rechts links Gestricke werden auf bekannte Weise aus einer Nadelreihe hergestellt. Die rechts links Ware hat zwei verschieden aussehende Warenseiten, der rechten und der linken Maschenseite. Ein rechts links Gestrick ist in Querrichtung wenig elastisch und sehr leicht.

Besonders vorteilhaft ist, daß die Warenränder einer solchen Wirkstruktur einrollen, wie dies beispielsweise bei der "Fluse" der Fall ist, was für die hier verlangte überlagerte Struktur und den Einsatzzweck von Vorteil ist. In diesem Fall kann die überlagerte Struktur durch das Wirkverfahren aufgeprägt werden. Alternativ und zusätzlich ist aber auch hier der Einsatz von Formgedächtnislegierungen möglich und sinnvoll.

Für die Herstellung derartiger gewirkter Strukturen können bekannte Wirkverfahren eingesetzt werden. Da die erfindungsgemäßen Implantate von ihren Ausmaßen her allerdings außerordentlich klein sind - beispielsweise mit einer Größe von 2 x 1 cm - hat es sich als vorteilhaft erwiesen, die Implantate im Rahmen eines herkömmlichen Gewirkes oder Gestrickes aus textilen, nicht metallischen Filamenten herzustellen, beispielsweise in Form einer aus den jeweiligen metallischen Filamenten gefertigten Randes, der entweder angearbeitet ist oder von dem das Gestrick oder Gewirke ausgeht. Die Anordnung des metallischen Teils des Gestrickes oder Gewirkes am Rand ist von Bedeutung, um den Einrolleffekt zu erzielen. Die nicht metallischen textilen Teile der Wirkstruktur werden dann anschließend durch Veraschen, chemisches Zerstören oder Auflösen in einem geeigneten Lösemittel entfernt.

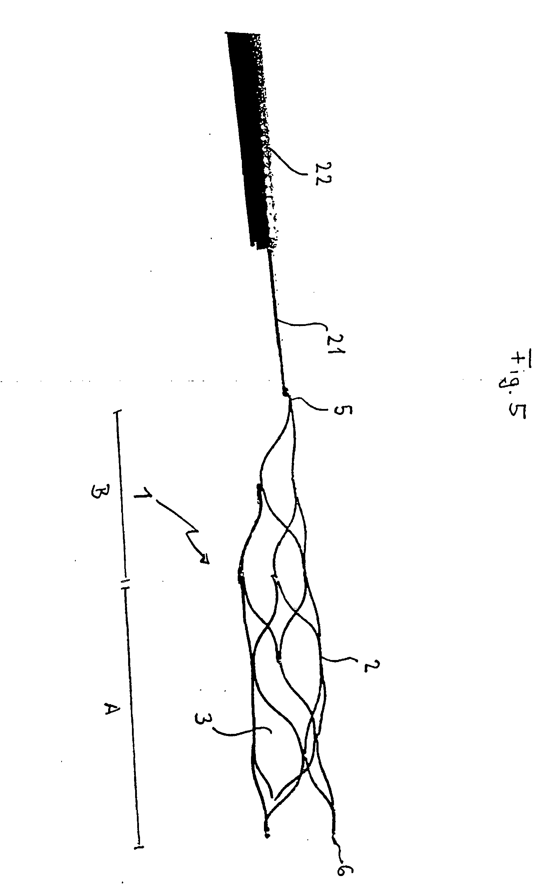

Figur 5 zeigt eine Kombination eines Fuhrungsdrahts 21 mit daran festgelegtem Implantat 1, daß aus einzelnen miteinander verschweißten Filamenten 2 besteht. Deutlich zu erkennen sind die distalen Enden 6 und der Verbindungspunkt 5, an dem die Filamente des Implantats in der sich verjüngenden Struktur zusammenlaufen und der gleichzeitig die Verbindung zum Führungsdraht 21 herstellt. Der Führungsdraht 21 wird in einen Mikrokatheter 22 geführt, bei dem es sich um ein herkömmliches Fabrikat handelt.

Durch Verschieben des Führungsdrahts 21 im Katheter 22 wird das Implantat 1 herausgeschoben bzw. hineingezogen. Beim Hinausschieben aus dem Mikrokatheter trachtet die Maschenstruktur danach, die ihr aufgegebene überlagerte Struktur einzunehmen, beim Hineinziehen rollt, bzw. faltet sich die Maschenstruktur entsprechend des räumlichen Gegebenheiten im Mikrokatheter wieder zusammen.

Aufgrund der Steifigkeit der Maschenstruktur kann das Implantat über den Führungsdraht 21 nahezu beliebig hin und her bewegt werden, bis es seine optimale Anordnung im Gefäßsystem gefunden hat.

Wie schon dargelegt, können herkömmliche Mikrokatheter eingesetzt werden. Der Vorteil des erfindungsgemäßen Implantats und der erfindungsgemäßen Kombination aus Implantat und Führungsdraht besteht allerdings darin, daß nach Setzen des Mikrokatheters mit einem üblichen Führungsdraht/Markersystem die erfindungsgemäße Kombination aus Führungsdraht 21 und Implantat 1 in den Mikrokatheter eingeschoben, durch diesen hindurch an den Einsatzort verbracht und dort ausgebracht werden kann. Alternativ ist es möglich, Führungsdraht 21 und Implantat 1 in einem zweiten Mikrokatheter kleineren Kalibers unterzubringen und mit diesem zweiten Mikrokatheter innerhalb des ersten gesetzten Mikrokatheters an den Einsatzort zu verschieben. In jedem Fall ergibt sich die einfache Führung des Implantats in beide Richtungen.

Figur 6 zeigt schematisch ein erfindungsgemäßes Implantat in seiner überlagerten und in seiner volumenreduzierten Form. In seiner expandierten Struktur gemäß Figur 6a bildet das Implantat 1 eine ringförmige Struktur mit leichter Überlappung der Ränder 7 und 8. Die Abbildung zeigt das Implantat 1 von seinem proximalen Ende her in der Draufsicht, mit dem Verbindungspunkt 5 in etwa gegenüberliegend zu den Rändern 7 und 8. Am Verbindungspunkt 5 schließt sich in der erfindungsgemäßen Kombination der Führungsdraht 21 an.

Figur 6a zeigt das gleiche Implantat in seiner volumenreduzierten Form, wie es sich beispielsweise eingerollt in einem Mikrokatheter anordnet. Im dargestellten Fall ergeben sich insgesamt zwei Wicklungen des eingerollten Implantats 1 mit dem Verbindungspunkt 5 an der proximalen Seite und den beiden Seitenrändern 7 und 8 als Anfangs- und Endpunkte der Rolle bzw. Spirale. Die Struktur wird durch den Mikrokatheter 22 in der volumenreduzierten Form gehalten; bei Herausschieben des Implantats 1 aus dem Mikrokatheter 22 springt dieses auf zur expandierten Form von Figur 6a, vergleichbar einer Spiralfeder.

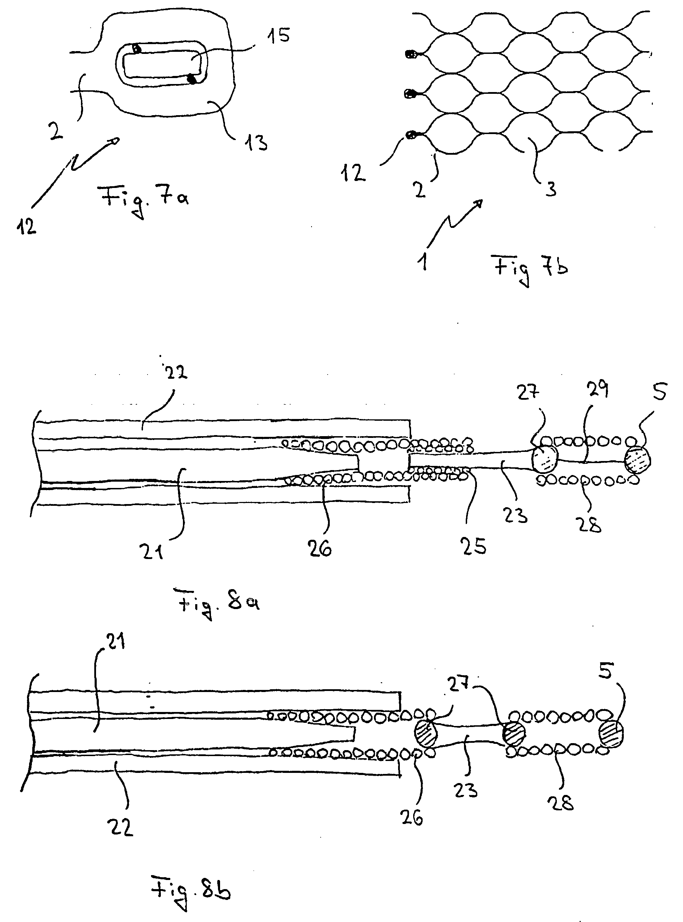

Figur 7a zeigt ein für die erfindungsgemäßen Implantate geeignetes Markerelement 12, wie es am distalen Ende des Implantats 1 zum Einsatz kommen kann. Das Markerelement 12 besteht aus einer "Öse" 13, die in ihrem Inneren flächeneben (in der Ebene des Implantats, ohne vorstehende Elemente) ein Markerplättchen 15 aus einem röntgendichten Material, beispielsweise Platin oder Platin-Iridium aufweist. Das Markerplättchen 15 ist über Laserschweißtechniken mit der umgebenen Implantatstruktur verbunden.

Figur 7b gibt ein Beispiel für die Anordnung der Markerelemente 12 am distalen Ende des Implantats 1 (s. Position 6 in Figur 1).

Figur 8 zeigt schematisch zwei Varianten einer Ablösestelle 8a und 8b, über die das erfindungsgemäße Implantat lösbar mit einem Führungsdraht verbunden ist. In beiden Fällen besteht die Ablösestelle 23 aus einem hantelförmigen Element, daß sich unter dem Einfluß von Strom bei Kontakt mit einem Elektrolyten auflöst. Das hantelförmige Element 23 gemäß Figur 8a weist an seinem proximalen (führungsdrahtseitigen) Ende eine Spiralstruktur 25 auf, die mit einer Verstärkungsspirale 26 des Führungsdrahts zusammenwirkt. Am distalen Ende befindet sich ein kugelförmiges Element 27, daß über Laserschweißtechnik mit einer Platinspirale 28 verbunden ist, die ihrerseits mit dem Verbindungspunkt 5 am proximalen Ende des Implantats verbunden ist. Die Platinspirale 28 dient zugleich als proximaler röntgendichter Marker des Implantats 1.

Zur Verstärkung der Verbindung zwischen dem kugelförmigen Element 27 und dem Verbindungspunkt 5 kann ein Verstärkungsdraht 29 sinnvoll sein. Alternativ kann aber auch die Platinspirale 28 konstruktiv so ausgelegt sein, daß sie den auf sie ausgeübten Zug- und Schubkräften Stand hält.

Für das Ablöseelement 23 kommt insbesondere Stahl in Frage, der unter dem Einfluß von Strom in einem Elektrolyten korrosionsanfällig ist. Zur Beschleunigung der Korrosion und Verminderung der Ablösezeit ist eine strukturelle oder chemische Schwächung der Hantel sinnvoll, beispielsweise durch Anschleifen oder eine thermische Behandlung.

Im allgemeinen hat der für den Elektrolyten zugängliche Bereich der Hantel 23 eine Länge von 0,1 bis 0,5 mm, insbesondere 0,3 mm.

Die Spiralstruktur 25 ist über Schweißpunkte sowohl mit dem hantelförmige Element 23 als auch mit der Verstärkungsspirale 26 des Führungsdrahts 21 verbunden. Der Führungsdraht 21 selbst ist im Mikrokatheter 22 verschiebbar gelagert.

Fig. 8b zeigt eine zweite Ausführungsform, die sich von der der Fig. 8a dadurch unterscheidet, daß das handelförmige Element 23 an beiden Enden ein kugelförmiges Element 27 aufweist, welche ihrerseits distal mit dem Verbindungspunkt 5 des Implantats und proximal mit dem Führungsdraht 21 über Spiralen 28 bzw. 26 verbunden sind.

Es versteht sich, daß andere Ablöseprinzipien verwand werden können, etwa solche, die auf mechanischen Prinzipien beruhen oder auf dem Abschmelzen von Kunststoffverbindungen basieren.

## Patentansprüche

1. Kombination eines medizinischen Implantats und eines Führungsdrahts, bei der das medizinische Implantat
eine Struktur (A) mit einer ersten Vielzahl von Maschen oder Waben, bei der die Struktur (A) ein proximales Ende und ein distales Ende aufweist, und
ferner eine sich verjüngende Struktur (B) mit einer Vielzahl von Maschen oder Waben umfasst, und die sich verjüngende Struktur (B) in Richtung des proximalen Endes der Struktur (A) gelegen ist und in die Struktur (A) übergeht,
und das medizinische Implantat in eine volumenreduzierte Form zum Einführen in einen Mikrokatheter (22) überführbar ist, wobei das medizinische Implantat Räder (7,8) aufweist, die sich jeweils in einer vom proximalen zum distalen Ende weisenden Richtung entlang der Struktur (A) erstrecken, und das medizinische Implantat aufgerollt werden kann und in der volumenreduzierten Form röhrenförmig ist und sich entlang einer Längsrichtung erstreckt,
**dadurch gekennzeichnet, dass**
die Ränder (7,8) in der volumenreduzierten Form einander zumindest nahe sind und sich im Randbereich überlappen, so dass das medizinische Implantat (1) derart aufgerollt ist, dass sie in einen Mikrokatheter eingeschoben und darin bewegt werden kann, und nach Freisetzung aus dem Mikrokatheter aufspringt und eine expandierte Form annimmt, wobei
das medizinische Implantat (1) weiter einen an einem proximalen Ende der sich verjüngenden Struktur (B) gelegenen Verbindungspunkt (5) umfasst, und die sich verjüngende Struktur (B) am Verbindungspunkt (5) zusammenläuft, und
ferner ein lösbar an den Verbindungspunkt (5) gekoppelter Führungsdraht (21) vorgesehen ist, welcher Führungsdraht geeignet ist, das Implantat durch den Katheter hindurch zum Einsatz zu schieben und in den Katheter hinein zurückzuziehen.

2. Kombination nach Anspruch 1, **dadurch gekennzeichnet, dass** sie zumindest teilweise aus einer Legierung mit Formgedächtniseigenschaften besteht.

3. Kombination nach Anspruch 1, **dadurch gekennzeichnet, dass** die sich verjüngende Struktur (B) in einer Platinspirale (28) zusammenläuft.

4. Kombination nach einem der vorstehenden Ansprüche,
**dadurch gekennzeichnet, dass** sie aus einer zu einem längs offenen Schlauch gerollten geschnittenen Folie besteht.

5. Kombination nach einem der Ansprüche 1 bis 4, **dadurch gekennzeichnet, dass** sie aus einer zu einem längs offenen Schlauch gerollten Streckmetallfolie besteht.

6. Kombination nach einem der Ansprüche 1 bis 3, **dadurch gekennzeichnet, dass** sie aus zu einer Maschenstruktur verschweißten einzelnen Filamenten (2) besteht.

7. Kombination nach einem der Ansprüche 1 bis 3, **dadurch gekennzeichnet, dass** sie aus einem Maschengeflecht einzelner Filamente (2) besteht.

8. Kombination nach Anspruch 6 oder 7, **dadurch gekennzeichnet, dass** die Filamente (2) seilartig aus einzelnen Litzen zusammengesetzt sind.

9. Kombination nach Anspruch 7, **dadurch gekennzeichnet, dass** das Maschengeflecht eine Wirkstruktur aufweist, die herstellungsbedingt zu eingerollten Rändern (7,8) führt.

10. Kombination nach Anspruch 9, **dadurch gekennzeichnet, dass** die Wirkstruktur eine "Fluse" ist.

11. Kombination nach einem der vorstehenden Ansprüche, **dadurch gekennzeichnet, dass** sie distal einen oder mehrere Marker (12) aufweist.

12. Kombination nach Anspruch 11, **dadurch gekennzeichnet, dass** der oder die Marker (12) endständig an Knotenpunkten der Stege oder Filamente (2) angeordnet sind.

13. Kombination gemäß Anspruch 1, **dadurch gekennzeichnet, dass** die Struktur (A) aus einem Metallrohr aufgebaut ist, das zum Ausbilden eines längs geöffneten Rohrs geschnitten wurde.

14. Kombination gemäß Anspruch 1, bei dem die Vielzahl der Maschen der Struktur (A) eine erste Größe aufweist, und die Vielzahl der Maschen der sich verjüngenden Struktur (B) eine zweite Größe aufweist, die größer als die erste Größe ist.

15. Kombination nach Anspruch 1, **dadurch gekennzeichnet, dass** der Führungsdraht (21) an seinem distalen Ende eine Platinspirale (26) aufweist, die über ein unter dem Einfluss von Strom korrodierbares Verbindungselement (23) mit das medizinische Implantat verbunden ist.

16. Kombination nach Anspruch 15, **dadurch gekennzeichnet, dass** das elektrisch korrodierbare Verbindungselement (23) zwischen einer distalen Platinspirale (26) des Führungsdrahts (21) und einer proximalen Platinspirale (28) des das medizinischen Implantats (1) angeordnet ist.

17. System zur Verwendung bei der Behandlung von Aneurysmen oder anderer vaskulärer Fehlbildungen mit
einem ersten Mikrokatheter (22);
einer Kombination nach einem der Ansprüche 1 bis 16, bei der das medizinische Implantat eine längs offene Struktur aufweist, die lösbar am distalen Ende des Führungsdrahtes (21) angeordnet ist.

18. System nach Anspruch 17, **dadurch gekennzeichnet, dass** die sich verjüngende Struktur (B) über ein elektrolytisch korrodierbares Element (23) mit dem Führungsdraht (21) verbunden ist.

19. System nach Anspruch 17 oder 18, **dadurch gekennzeichnet, dass** es zusätzlich einen zweiten Mikrokatheter aufweist, der dazu ausgelegt und bestimmt ist, den Führungsdraht (21) mit der Struktur (A) und der sich verjüngende Struktur (B) in sich verschiebbar aufzunehmen und sie durch den ersten Mikrokatheter zum Einsatzort zu führen.

20. System nach einem der Ansprüche 17 bis 19, **dadurch gekennzeichnet, dass** es zusätzliche Einrichtungen zur Durchführung der Elektrokorrosion des Verbindungselements aufweist.

21. System nach einem der Ansprüche 18 bis 20, **dadurch gekennzeichnet, dass** das medizinische Implantat (1) seine ihm aufgegebene expandierte Form einnehmen kann, soweit es die Dimensionen der umgebenden Gefäße zulassen.

## Claims

1. Combination of a medical implant and a guide wire, in which the medical implant comprises a structure (A) with a first plurality of loops or honeycombs, in which the structure (A) has a proximal end and a distal end, and furthermore a tapering structure (B) with a plurality of loops and honeycombs, and the tapering structure (B) is placed in the direction of the proximal end of the structure (A) and merges into structure (A), and the medical implant can be transferred to a volume-reduced form for introduction into a microcatheter (22), wherein the medical implant has edges (7, 8) which extend in each case in a direction along structure (A) pointing from the proximal to the distal end, and the medical implant maybe rolled up and is tubular in the volume-reduced form and extends along a longitudinal direction, **characterised in that** the edges (7, 8) in the volume-reduced form are at least close to one another and overlap in the edge region so that the medical implant (1) is rolled up such that it maybe put into a microcatheter and moved therein, and after release from the microcatheter uncurls and adopts an expanded form, wherein the medical implant (1) furthermore comprises a connecting point (5) placed on a proximal end of the tapering structure (B), and the tapering structure (B) converges at the connecting point (5), and furthermore a guide wire (21) coupled releasably at the connecting point (5) is provided, which guide wire is suitable to push the implant though the catheter for use and to retract it into the catheter.

2. Combination according to claim 1, **characterised in that** it consists at least partly of an alloy with shape-memory properties.

3. Combination according to claim 1, **characterised in that** the tapering structure (B) converges into a platinum coil (28).

4. Combination according to one of the preceding claims, **characterised in that** it consists of a cut foil rolled to form a longitudinally open tube.

5. Combination according to one of claims 1 to 4, **characterised in that** it consists of an expanded metal foil rolled to form a longitudinally open tube.

6. Combination according to one of claims 1 to 3, **characterised in that** it consists of individual filaments (2) welded to form a loop structure.

7. Combination according to one of claims 1 to 3, **characterised in that** it consists of a loop network of individual filaments (2).

8. Combination according to claim 6 or 7, **characterised in that** the filaments (2) are composed like rope of individual braids.

9. Combination according to claim 7, **characterised in that** the loop network has a knitted structure which leads, depending on production, to rolled-up edges (7, 8).

10. Combination according to claim 9, **characterised in that** the knitted structure is a "bobble".

11. Combination according to one of the preceding claims, **characterised in that** it has one or more markers (12) distally.

12. Combination according to claim 11, **characterised in that** the marker or markers (12) are arranged terminally at node points of the bars or filaments (2).

13. Combination according to claim 1, **characterised in that** the structure (A) is constructed from a metal tube which has been cut to form a longitudinally opened tube.

14. Combination according to claim 1, in which the plurality of loops of structure (A) have a first size, and the plurality of loops of the tapering structure (B) have a second size which is greater than the first size.

15. Combination according to claim 1, **characterised in that** the guide wire (21) has at its distal end a platinum coil (26) which is connected to the medical implant via a connecting element (23) which can be corroded under the influence of current.

16. Combination according to claim 15, **characterised in that** the electrically corrodible connecting element (23) is arranged between a distal platinum coil (26) of the guide wire (21) and a proximal platinum coil (28) of the medical implant (1).

17. System for use in the treatment of aneurysms or other vascular abnormalities having a first microcatheter (22); a combination according to one of claims 1 to 16, in which the medical implant has a longitudinally open structure which is arranged releasably on the distal end of the guide wire (21).

18. System according to claim 17, **characterised in that** the tapering structure (B) is connected to the guide wire (21) via an electrolytically corrodible element (23).

19. System according to claim 17 or 18, **characterised in that** it additionally has a second microcatheter which is designed and intended to accommodate the guide wire (21) with the structure (A) and the tapering structure (B) to be displaceable in itself and to guide it through the first microcatheter to the site of use.

20. System according to one of claims 17 to 19, **characterised in that** it has additional devices for carrying out electro-corrosion of the connecting element.

21. System according to one of claims 18 to 20, **characterised in that** the medical implant (1) may assume its expanded form given to it provided the dimensions of the surrounding vessels allow it.

## Revendications

1. Combinaison d'un implant médical et d'un fil de guidage, dans laquelle l'implant médical
comprend une structure (A) avec une première pluralité de mailles ou d'alvéoles, dans laquelle la structure (A) présente une extrémité proximale et une extrémité distale, et
comprend en outre une structure conique (B) avec une pluralité de mailles ou d'alvéoles et la structure conique (B) est posée en direction de l'extrémité proximale de la structure (A) et effectue une transition vers la structure (A),
et l'implant médical peut se rétrécir vers une forme à volume réduite pour , l'introduction dans un micro-cathéter (22), l'implant médical présentant des bords (7, 8) qui s'étendent chacun dans une direction de l'extrémité proximale vers l'extrémité distale le long de la structure (A) et l'implant médical peut être enroulé et se présente, dans la forme à volume réduit, sous la forme d'un tube et s'étend le long d'une direction longitudinale,
**caractérisée en ce que**
les bords (7, 8), dans la forme à volume réduit, sont au moins proches les uns des autres et se chevauchent au niveau du bord, de façon à ce que l'implant médical (1) soit enroulé de façon à ce qu'il puisse être inséré dans un micro-cathéter et y être déplacé à l'intérieur et, après la libération, s'éjecte hors du micro-cathéter et adopte une forme dilatée,
l'implant médical (1) comprenant en outre un point de liaison (5) disposé à une extrémité proximale de la structure conique (B), et la structure conique (B) se rétrécit au niveau du point de liaison, et
un fil de guidage (21) couplé de manière amovible au point de liaison (5) est prévu, ce fil de guidage étant conçu pour pousser l'implant à travers le cathéter pour l'insérer et le retirer à intérieur du cathéter.

2. Combinaison selon la revendication 1, **caractérisée en ce qu'**elle est constituée au moins partiellement d'un alliage à mémoire de forme.

3. Combinaison selon la revendication 1, **caractérisée en ce que** la structure conique (B) se rétrécit en une spirale de platine (28).

4. Combinaison selon l'une des revendications précédentes, **caractérisée en ce qu'**elle est constituée d'un film découpé roulé en un tube ouvert dans le sens de la longueur.

5. Combinaison selon l'une des revendications 1 à 4,
**caractérisée en ce qu'**elle est constituée d'un film métallique roulé en un tube ouvert dans le sens de la longueur.

6. Combinaison selon l'une des revendications 1 à 3, **caractérisée en ce qu'**elle est constituée de filaments (2) séparés soudés en une structure à mailles.

7. Combinaison selon l'une des revendications 1 à 3,
**caractérisée en ce qu'**elle est constituée d'une tresse à mailles de filaments (2) séparés.

8. Combinaison selon la revendication 6 ou 7,
**caractérisée en ce que** les filaments (2) sont constitués de torons séparés.

9. Combinaison selon la revendication 7, **caractérisée en ce que** le treillis de mailles présente une structure active qui conduit, grâce à la construction, vers des bords enroulés (7, 8).

10. Combinaison selon la revendication 9, **caractérisée en ce que** la structure active est une « peluche ».

11. Combinaison selon l'une des revendications précédentes, **caractérisée en ce qu'**elle comprend, distalement, un ou plusieurs marqueurs (12).

12. Combinaison selon la revendication 11,
**caractérisée en ce que** le ou les marqueurs (12) sont disposés aux extrémités, au niveau des points nodaux des nervures ou des filaments (2).

13. Combinaison selon la revendication 1, **caractérisée en ce que** la structure (A) est constituée d'un tube métallique qui a été découpé pour la formation d'un tube ouvert dans le sens de la longueur.

14. Combinaison selon la revendication 1, dans laquelle la pluralité de mailles de la structure (A) présente une première dimension et la pluralité de mailles de la structure conique (B) présente une deuxième dimension supérieure à la première dimension.

15. Combinaison selon la revendication 1, **caractérisée en ce que** le fil de guidage (21) présente, au niveau de son extrémité distale, une spirale de platine (26), reliée à l'implant médical par l'intermédiaire d'un élément de liaison (23) pouvant être corrodé par l'action d'un courant.

16. Combinaison selon la revendication 15,
**caractérisée en ce que** l'élément de liaison (23) pouvant être corrodé par l'action d'un courant est disposé entre une spirale de platine distale (26) du fil de guidage (21) et une spirale de platine proximale (28) de l'implant médical(1).

17. Système permettant une utilisation pour le traitement d'anévrismes ou d'autres malformations vasculaires, avec
un premier micro-cathéter (22) ;
une combinaison selon l'une des revendications 1 à 16, dans laquelle l'implant médical présente une structure ouverte dans le sens de la longueur, qui est disposée de manière amovible à l'extrémité distale du fil de guidage (21).

18. Système selon la revendication 17, **caractérisé en ce que** la structure conique (B) est relié au fil de guidage (21) par l'intermédiaire d'un élément (23) pouvant être corrodé de manière électrolytique.

19. Système selon la revendication 17 ou 18,
**caractérisé en ce qu'**il comprend en outre un deuxième micro-cathéter conçu pour loger de manière coulissante le fil de guidage (21) avec la structure (A) et la structure conique (B) et pour les guider à travers le premier micro-cathéter vers le lieu d'insertion.

20. Système selon l'une des revendications 17 à 19,
**caractérisé en ce qu'**il comprend des dispositifs supplémentaires pour la réalisation d'une corrosion électrolytique de l'élément de liaison.

21. Système selon l'une des revendications 18 à 20,
**caractérisé en ce que** l'implant médical (1) peut prendre sa forme dilatée dès que les dimensions des vaisseaux environnants le permettent.
